# EUROPEAN PATENT APPLICATION

(11) **EP 1 360 959 A1**
(43) Date of publication of application: **12.11.2003**
(21) Application number: 02090170.8
(22) Date of filing: 10.05.2002
(51) Int. Cl.: A61K 31/352, A61P 35/00, A61P 19/02, A61P 17/06, A61P 27/02, A61P 19/10

(54) **Use of 8-prenylflavanones for anti-angiogenesis therapy and for fibrinolytic therapy**

(71) Applicant: Schering Aktiengesellschaft, 13353 Berlin (DE)
(72) Inventor: Schleuning, Wolf-Dieter, 13467 Berlin (DE); Pepper, Michael, 1245 Collonge-Bellerive (CH)

(57) **Abstract**

The present invention provides the use of 8-Prenylflavanones of the general formula I: 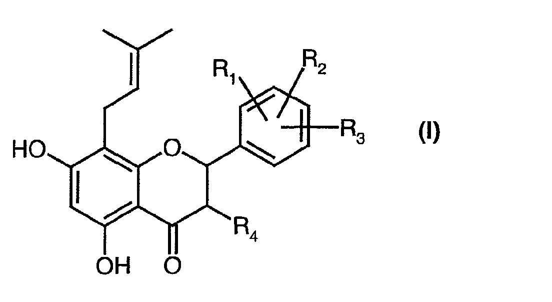 wherein
- R₁: designates a hydrogen atom, a hydroxyl group in position 2', 3' or 4', a methoxy group in position 2', 3' or 4' or an ethoxy group in position 3' or 4',
- R₂: designates a hydrogen atom, a hydroxyl group in position 3', 4', 5' or 6', a methoxy group in position 3' or 4' or an ethoxy group in position 5',
- R₃: designates a hydrogen atom, a hydroxyl group in position 4', 5' or 6' or a methoxy group in position 4', 5' or 6',
- R₄: designates a hydrogen atom or a hydroxyl group,
for the preparation of a pharmaceutical composition for the treatment or prevention of angiogenesis-related diseases and for the treatment or prevention of thrombotic disorders.

## Description

The invention relates to the use of 8-Prenylflavanones in a pharmaceutical composition.

8-Prenylflavanones are found in a variety of plants. One member of this group of compounds is 8-Prenylnaringenin (5,7-Dihydroxy-2-(4-hydroxyphenyl)-8-(3-methylbut-2-enyl)-chroman-4-on) which occurs in e.g. *Helichrysum hypocephalum* (Bohlmann, F. et al. 1979, Phytochemistry 18, 1246-1247) and citrus plants (Ito, C. et al. 1988, Chem. Pharm. Bull. 36, 3292-3295). It has the following structure:

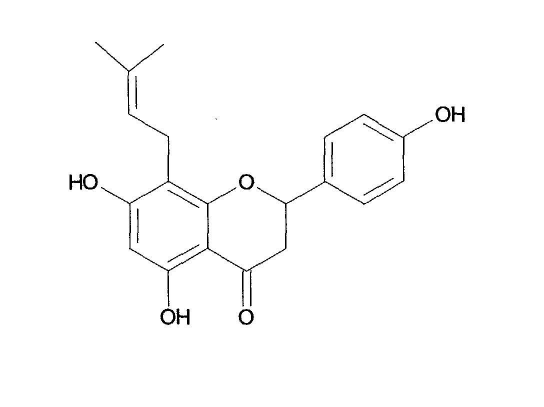

It has been shown to have estrogenic activity (Kitaoka, M. JP 08-165238).

Angiogenesis, the formation of new capillary blood vessels, is required for tissue growth, wound healing and female reproductive function, and is an important component of hemangiomas and the process of ocular neovascularization (reviewed by Pepper, 1997, Arterioscler. Thromb. Vasc. Biol. 17: 605-619). It is also essential for the growth and persistence of solid tumors and their metastases. For solid tumors to grow beyond a critical size, they must recruit endothelial cells from the surrounding stroma to form their own endogenous microcirculation (Folkman, 1974). To stimulate angiogenesis, the production of positive regulators is upregulated. Positive regulators of angiogenesis include members of the vascular endothelial growth factor (VEGF) and basic fibroblast growth factor (bFGF) families. Endogenous negative regulators of angiogenesis, i.e. inhibitors of angiogenesis, include angiostatin, endostatin, thrombospondin, interferons, platelet factor IV, 16kDa N-terminal fragment of prolactin, IL-12, gro-β, proliferin-related protein, protease inhibitors, 2-methoxyestradiol and soluble cytokine. Most of these compounds are peptides or proteins.

For the treatment of angiogenesis-related diseases, it is desirable to have a low molecular weight compound which inhibits angiogenesis.

Fibrinolysis is controlled by the expression of plasminogen activator (tPA) and plasminogen activator inhibitor-1 (PAI-1). tPA or other plasminogen activators are used for the therapy of acute myocardial infarction. This therapy is, however, not applicable for the prevention or longterm treatment of thrombotic diseases. In this case, an endogenous increase of the fibrinolytic activity would be desirable. This could be achieved by increasing the plasminogen activator activity and by deceasing the activity of PAI-1. Substances have been described which either increase both activities (e.g. VEGF, Pepper et al. 1991 Arterioscler. Thromb. Vasc. Biol. 17: 605-619; Mandriota et al. 1995, J. Biol. Chem. 270: 9709-9716 ) or have only very little effect on tPA although they increase the urokinase-type plasminogen activator (e.g. bFGF, Pepper et al. 1990, J. Cell Biol. 111: 743-755).
Therefore, the problem is to find a substance which will increase the endogenous tPA activity and decrease the PAI-1 activity.

The present invention solves the two problems by providing the use of 8-Prenylflavanones of the general formula I: 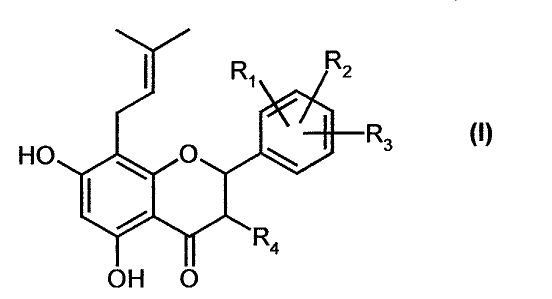 wherein
- R₁: designates a hydrogen atom, a hydroxyl group in position 2', 3' or 4', a methoxy group in position 2', 3' or 4' or an ethoxy group in position 3' or 4',
- R₂: designates a hydrogen atom, a hydroxyl group in position 3', 4', 5' or 6', a methoxy group in position 3' or 4' or an ethoxy group in position 5',
- R₃: designates a hydrogen atom, a hydroxyl group in position 4', 5' or 6' or a methoxy group in position 4', 5' or 6',
- R₄: designates a hydrogen atom or a hydroxyl group,
for the preparation of a pharmaceutical composition for the treatment and prevention of angiogenesis-related diseases and for the treatment and prevention of thrombotic disorders. The preferred compound of formula I is 8-Prenylnaringenin wherein R1, R2 and R4 designate a hydrogen atom and R3 designates a hydroxyl group in position 4'.

The inhibitory effect of 8-Prenylflavanones on angiogenesis is surprising since so far 8-Prenylflavanones have only been known to have estrogenic activity. 8-Prenylnaringenin inhibits angiogenesis induced by various positive regulators, e.g. bFGF and VEGF and also the synergistic effect of bFGF and VEGF in combination.

One or more 8-Prenylflavanones of the general formula I can be used for the preparation of a pharmaceutical composition for the prevention and treatment of angiogenesis-related diseases. Angiogenesis-related diseases are e.g. angiogenesis-dependent cancers, benign tumors, rheumatoid arthritis, psoriasis, ocular angiogenesis disease, plaque neovascularization, atherosclerosis and scleroderma.

The 8-Prenylflavanones of the general formula I increase the expression of tPA, measured by determining the amount of the corresponding mRNA, and the activity of tPA, measured by zymography. The amount of RNA can be determined by a Northern blot analysis as described in example 6 and the zymography can be performed as described in example 5. In addition, the 8-Prenylflavanones of the general formula I decrease the activity of PAI-1, measured by reverse zymography (see example 5) and increases the expression of uPA, measured by determining the amount of the corresponding mRNA, and the activity of uPA. Genistein, an structurally unrelated phytoestrogen, has previously been shown to decrease uPA (Fotsis et al. 1993, Cancer Res. 57: 2916-2921).

An object of the invention is the use of one or more 8-Prenylflavanones of the general formula I for the preparation of a pharmaceutical composition for the treatment and prevention of thrombotic disorders. Thrombotic disorders are e.g. coronary artery thrombosis (acute mycardial infarction), venous thrombosis, cerebral artery thrombosis (ischemic stroke), thrombangitis obliterans (Bürger's disease), arteriitis temporalis (Horton's disease), arteriitis Takayashu and APC-resistance.

In a further object, the present invention concerns a pharmaceutical composition comprising one or more 8-Prenylflavanones and at least one pharmaceutically acceptable excipient for the treatment of angiogenesis-related diseases and for the treatment and prevention of thrombotic disorders. These excipients may be carriers, diluents, absorption enhancers, preservatives, buffers, agents for adjusting the osmotic pressure, tablet disintegrating agents and other ingredients which are conventionally used in the art. Examples of solid carriers are magnesium carbonate, magnesium stearate, dextrin, lactose, sugar, talc, gelatin, pectin, tragacanth, methyl cellulose, sodium carboxymethyl cellulose, low melting waxes and cocoa butter. Liquid compositions include sterile solutions, suspensions and emulsions. A composition for transdermal administration of 8-Prenylnaringenin may be provided in the form of a patch and a composition for nasal administration may be provided in the form of a nasal spray in liquid or powder form.

Usually, not more than 1000 mg, preferably not more than 100 mg, and in some preferred instances not more than 10 mg of one or more 8-Prenylflavanones is to be administered to a human per day.

### Examples

### Example 1: Synthesis of 8-Prenylflavanones

8-Prenylflavanones are synthesized by the methods described in JP 08-165238. 8-Prenylnaringenin was synthesized as described in Satoshi Tahara, Phytochemistry 36, 1261-1271, 1994.

### Example 2: Cell culture

Bovine adrenal cortex-derived microvascular endothelial cells (BME cells, Furie et al., 1984, J. Cell Biol. 98, 1033-1041) were grown in minimal essential medium, alpha modification (α-MEM - Gibco BRL), supplemented with heat-inactivated donor calf serum (DCS - Flow Laboratories), penicillin (110U/ml) and streptomycin (110µg/ml). Bovine aortic endothelial (BAE) cells, isolated from scrapings of adult bovine thoracic aortas and cloned by limiting dilution as previously described (Pepper et al., Am J. Physiol. 262: C1246-C1257, 1992), were cultured in low glucose Dulbecco's modified minimal essential medium (DMEM, Gibco) supplemented with 10% DCS and antibiotics. Cell lines were maintained in 1.5% gelatin-coated tissue culture flasks (Falcon Labware, Becton-Dickinson Company) and subcultured at a split ratio of 1/3 or 1/4. The endothelial nature of the cells has previously been confirmed by Dil-Ac-LDL (Paesel and Lorei) uptake and immunostaining with a rabbit polyclonal antiserum against human von Willebrand factor (Nordic Immunology).

### Example 3:

### 8-Prenylnaringenin inhibits BME and BAE cell in vitro angiogenesis

bFGF and VEGF induce BME and BAE cells to invade three-dimensional collagen gels, and this is accompanied by the formation of capillary like tubular structures (Montesano et al., Proc. Natl. Acad. Sci. USA 83: 7297-7301, 1986; Pepper et al., Biochem. Biophys. Res. Comm. 189: 824-831, 1992; Pepper et al., Exp. Cell Res. 204: 356-363, 1993, Pepper et al., J. Cell Sci. 108: 73-83, 1995). In addition, bFGF and VEGF induce a synergistic invasive response in BME and BAE cells (Pepper et al., 1992, Biochem. Biophys. Res. Commun. 189: 824-831; Pepper et al 1995, J. Cell Sci. 108: 73-83).

### In vitro angiogenesis assay:

Three-dimensional collagen gels were prepared as described (Montesano et al., Cell 42: 469-477, 1985): 8 volumes of a solution of type I collagen from rat tail tendons (approximately 1.5mg/ml) were mixed with 1 volume of 10x minimal essential medium and 1 volume of sodium bicarbonate (11.76mg/ml) on ice, dispensed into 18mm tissue culture wells, and allowed to gel at 37ºC for 10 minutes. BME cells were then seeded onto the collagen gels at 1.0 x 10⁵ cells/well in 500µl α-MEM supplemented with 5% DCS and were grown to confluence (3-4 days), at which point serum was reduced to 2% and treatment with bFGF (155 amino acid form) and/or VEGF (165-amino acid homodimeric species-VEGF₁₆₅ from Peprotech) and 8-Prenylnaringenin was begun. Medium and treatments were renewed after 2 and 4 days. Three randomly selected fields measuring 1.0 mm x 1.4 mm were photographed in each well at a single level beneath the surface monolayer by phase contrast microscopy using a Nikon Diaphot TMD inverted photomicroscope. Invasion was quantitated by determining the total additive length of all cellular structures which had penetrated beneath the surface monolayer either as apparently single cells or in the form of cell cords (Pepper et al., Biochem. Biophys. Res. Comm. 189: 824-831, 1992). Results are expressed as the mean total additive sprout length in µm ± s.e.m., and are from three separate experiments per condition. In the case of (±)-8-Prenylnaringenin, cells treated with bFGF (10ng/ml) or bFGF + VEGF were photographed after 2-3 days, whereas cells treated with VEGF (30ng/ml) alone were photographed after 4-7 days. It has previously been shown that the kinetics of VEGF-induced invasion are slower than those for bFGF or bFGF + VEGF (Pepper et al., J. Cell Physiol. 177: 439-452, 1998).

### Results:

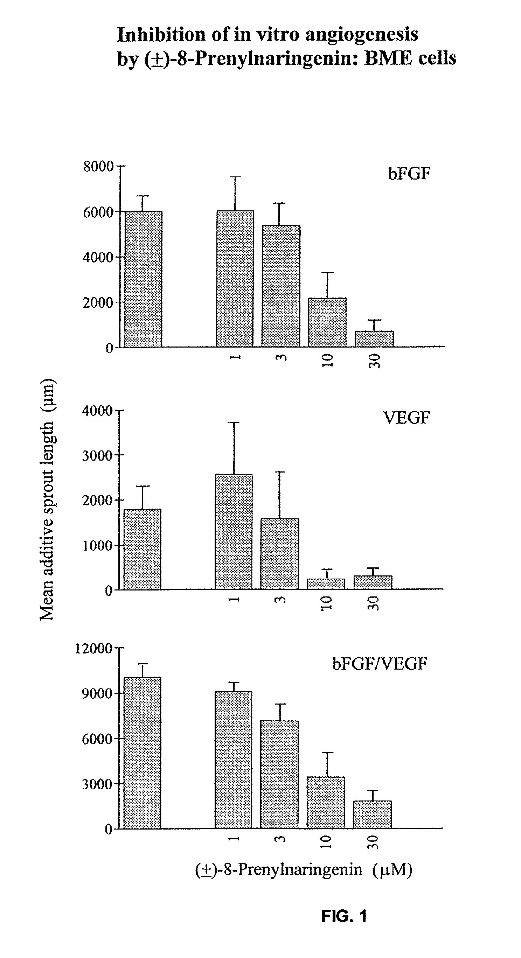

(±)-8-Prenylnaringenin inhibits in vitro angiogenesis induced by bFGF, VEGF or a combination of the bFGF and VEGF, in a dose-dependent manner (Figure 1). A quantitative analysis revealed that 8-Prenylnaringenin inhibited BME cell invasion with an IC₅₀ of between 3-10µM, irrespective of the angiogenic stimulus (Figure 1).

### Example 4: 8-Prenylnaringenin inhibits BME cell proliferation

### Proliferation assay:

BME cells were seeded into gelatin-coated wells of 24 well plates at 10'000 cells per well in α-MEM supplemented with 5% DCS. 24 hours later, fresh medium was added together with 8-Prenylnaringenin at the indicated concentrations or TGF-β1 (1ng/ml), and bFGF (10ng/ml) was added to half the wells. 2 days later, medium and compounds were renewed, and after a further 3 days (i.e. 5 day assay), cells were trypsinized and counted in a FACScan Analyzer. Results are shown as % inhibition calculated from the mean of duplicate wells.

### Results:

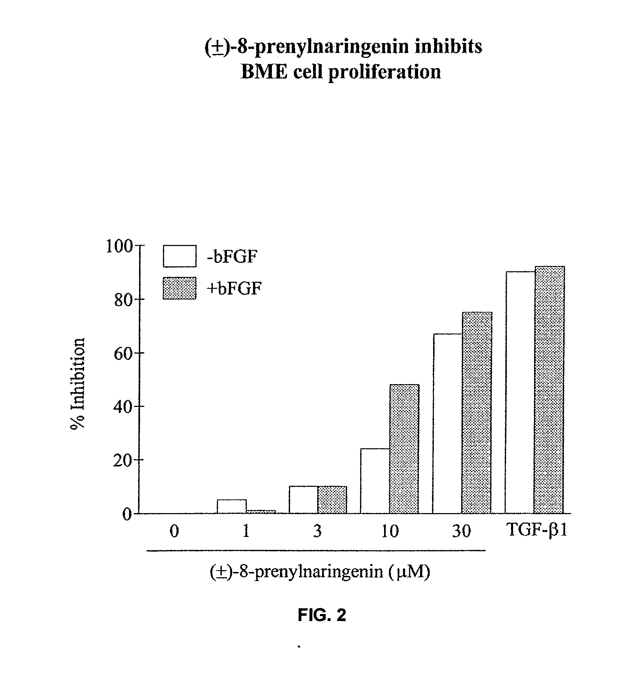

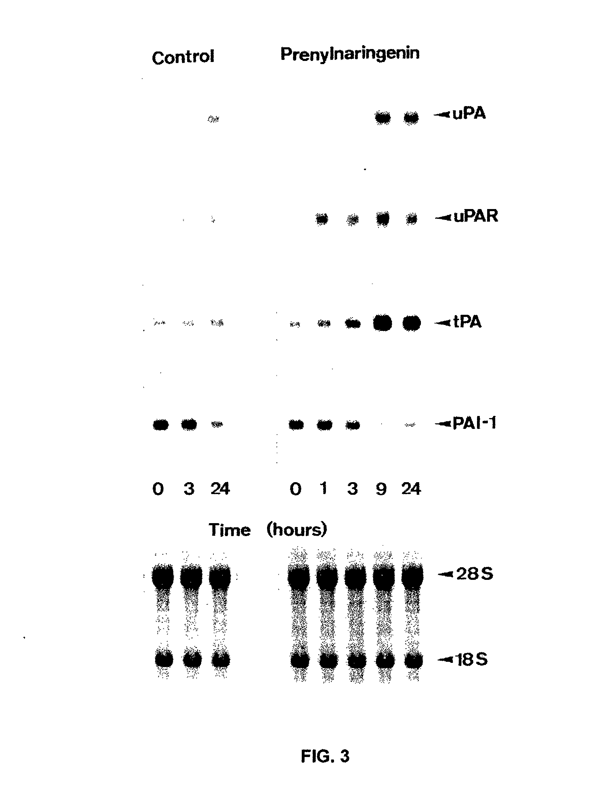

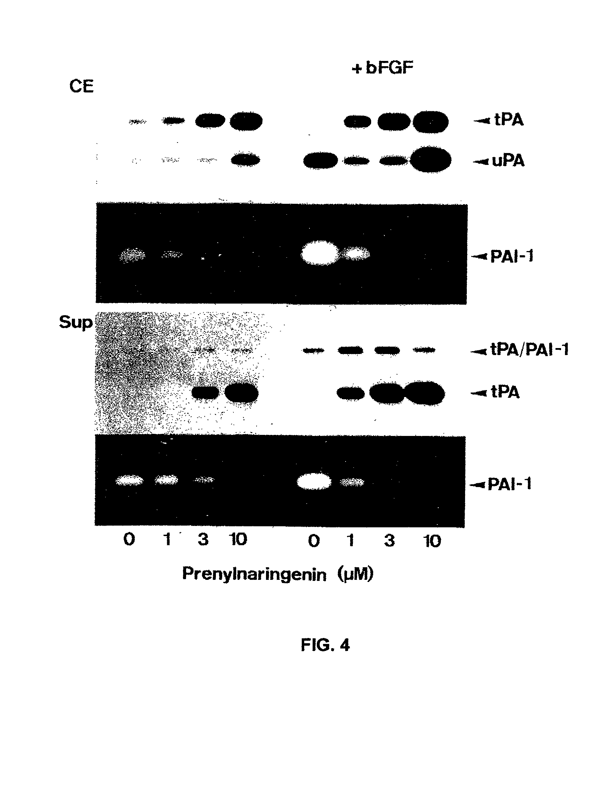

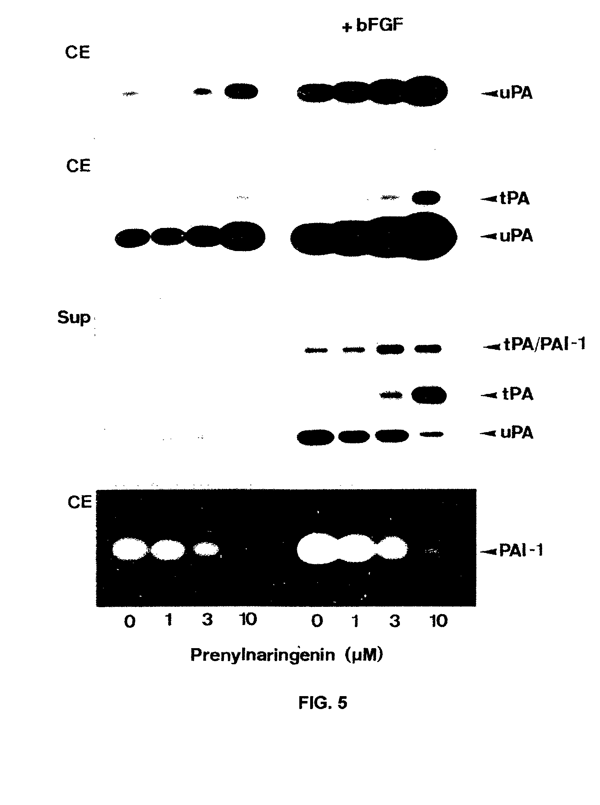

In the 5 day proliferation assay in the presence of 5% DCS, 8-Prenylnaringenin reduced BME cell number in a dose dependent manner (Figure 2). This effect was observed whether or not bFGF was co-added to the medium. Inhibition occurred with an IC₅₀ of about 10µM, and a maximal inhibitory effect was observed with 30µM 8-Prenylnaringenin (67 and 75% inhibition in the absence and presence of bFGF respectively). For comparison, TGF-β1 inhibited BME cell proliferation by 90 and 92% in the absence or presence of bFGF respectively (Figure 2).

### Example 5: Zymography and reverse zymography

### Assay:

Confluent monolayers of BME or BAE cells in 35mm gelatin-coated tissue culture dishes were washed twice with serum-free medium, and were treated with (±)-8-Prenylnaringenin in the absence or presence of bFGF or VEGF in serum-free medium containing Trasylol (200 Kallikrein Inhibitory Units/ml). 15 hours later, cell extracts were prepared and analyzed by zymography and reverse zymography as previously described (Vassalli et al., 1984, J. Exp. Med. 159: 1653-1668).

### Results:

By zymography, we observed that (±)-8-Prenylnaringenin induced a dose-dependent increase in uPA and tPA activity in BME cells (Figure 1). This was accompanied by a reduction in PAI-1 activity as detected by reverse zymography, which in all likelihood is due to sequestering of PAI-1 into a tPA/PAI-1 complex. (Addition of Trasylol to the serum-free culture medium retains uPA and tPA in a single-chain form; however, unlike uPA, tPA in this form is able to bind to PAI-1, a situation which cannot be avoided under the culture conditions employed). We have previously reported that bFGF markedly increases uPA with very little effect on tPA in BME cells (Pepper et al., 1990, J. Cell Biol. 111: 743-755). Here we have observed that bFGF potentiates the induction of tPA activity by (±)-8-Prenylnaringenin, with a variable concentration-dependent effect on uPA (Figure 9). We have previously reported that VEGF induces uPA, uPAR, tPA and PAI-1 in BME cells (Pepper et al., 1991, Arterioscler. Thromb. Vasc. Biol. 17: 605-619.; Mandriota et al., 1995, J. Biol. Chem. 270: 9709-9716). In the present studies we have observed that VEGF and (±)-8-Prenylnaringenin synergize in the induction of tPA activity in BME cells (data not shown). In the experiments described above, in which cells were incubated for 15 hours in the absence of serum for zymographic and reverse zymographic analysis, frank toxicity was observed with 30µM (±)-8-Prenylnaringenin. This is in contrast to toxicity seen at 100µM when (±)-8-Prenylnaringenin was added to BME cells in the presence of 2% serum.
By zymography, both uPA and tPA activity were increased in BAE cells by (±)-8-Prenylnaringenin; this was accompanied by a decrease in PAI-1, as detected by reverse zymography (Figure 10). As described above for BME cells, the reduction of PAI-1 activity is in all likelihood due to sequesterinh of PAI-1 in a tPA/PAI-1 complex. We have previously reported that bFGF increases uPA and tPA in BAE cells (Mandriota et al., 1995, J. Biol. Chem. 270: 9709-9716). Here we show that in BAE cells, bFGF potentiates the induction of tPA activity by (±)-8-Prenylnaringenin, and that (±)-8-Prenylnaringenin reduces bFGF-induced uPA activity (Figure 10).

### Example 6: RNA preparation, in vitro transcription, and Northern blot hybridization

### Method:

(±)-8-Prenylnaringenin or genistein were added to confluent BME cell monolayers to which fresh medium containing 5% DCS had been added 24 hours previously. Total cellular RNA was prepared after the indicated times using Trizol reagent (Life Technologies AG, Basel, Switzrland) according to the manufacturer's instructions. Northern blots, UV cross-linking and methylene blue staining of filters, in vitro transcription, hybridization and post-hybridization washes were as previously described (Pepper et al., 1990, J. Cell Biol. 111: 743-755). [³²P]-labelled cRNA probes were prepared from bovine urokinase-type plasminogen activator (u-PA) (Krätzschmar et al., 1993, Gene 125: 177-183), bovine u-PA receptor (u-PAr) (Krätzschmar et al., 1993, Gene 125: 177-183), human tissue-type PA (t-PA) (Fisher et al., 1985, J. Biol. Chem. 260: 11223-11230) and bovine PA inhibitor-1 (PAI-1) (Pepper et al., 1990, J. Cell Biol. 111: 743-755) cDNAs as previously described (Pepper et al., 1990, J. Cell Biol. 111: 743-755). Filters were exposed to Kodak X-OMAT XAR-5 Scientific Imaging Film (Eastman Kodak Co., Rochester, NY). Autoradiograms were scanned and quantitated using ImageQuant (Molecular Dynamics, Sunnyvale, CA).

### Results:

By northern blot analysis, we found that (±)-8-Prenylnaringenin increased steady state levels of uPA, uPAR and tPA mRNAs in BME cells (Figures 7 and 8). The maximal effect of (±)-8-Prenylnaringenin was observed after 9 hours for uPA (7.5-fold increase), uPAR (6.4-fold increase) and tPA (9.6-fold increase). (±)-8-Prenylnaringenin had no significant effect on the levels of PAI-1 mRNA.

In contrast to BME cells, (±)-8-Prenylnaringenin had virtually no effect on uPA or uPAR mRNA levels in BAE cells, but did induce a 4.6-fold increase in tPA levels after 24 hours (Figure 8).

### Description of the figures

### Figure 1

8-Prenylnaringenin inhibits angiogenesis *in vitro*. BME cells were treated for 3 days with bFGF (10ng/ml), VEGF (30ng/ml) or a combination of bFGF and VEGF, either in the absence or presence of 8-Prenylnaringenin at the indicated concentrations. Results are expressed as the mean additive sprout length ± s.e.m. from at least three separate experiments per condition.

### Figure 2

8-Prenylnaringenin reduces endothelial cell number in a 5 day proliferation assay. BME cells were seeded into 24 well plates at 10'000 cells per well in medium containing 5% DCS. 24 hours later, cells were treated with 8-Prenylnaringenin at the indicated concentrations or with TGF-β1 (1ng/ml), and bFGF (10ng/ml) was added to half the wells. Results are shown as % inhibition calculated from the mean of duplicate wells.

### Figure 3

(±)-8-prenylnaringenin increases steady-state levels of uPA, uPAR and tPA mRNAs in BME cells.

Replicate filters containing total cellular RNA (5 µg/lane) prepared from confluent monolayers of BME cells incubated in the absence (Control) or presence of 10µM (±)-8-prenylnaringenin for the indicated times, were hybridized with ³²P-labelled cRNA probes. RNA integrity and uniformity of loading were determined by staining the filters with methylene blue after transfer and cross-linking (lower panel of the figure); 28S and 18S ribosomal RNAs are shown.

### Figure 4

Effect of (±)-8-prenylnaringenin on uPA, tPA and PAI-1 activity in BME cells.

Cell extracts (CE) and culture supernatants (Sup) prepared from BME cells incubated with (±)-8-prenylnaringenin at the indicated concentrations in the absence or presence of bFGF (10ng/ml) for 15 hours, were subjected to zymography (uPA, tPA and tPA/PAI-1 complex activities) and reverse zymography (PAI-1 activity)

### Figure 5

Effect of (±)-8-prenylnaringenin on uPA, tPA and PAI-1 activity in BAE cells.

Cell extracts (CE) and culture supernatants (Sup) prepared from BAE cells incubated with (±)-8-prenylnaringenin at the indicated concentrations in the absence or presence of bFGF (3ng/ml) for 15 hours, were subjected to zymography (uPA, tPA and tPA/PAI-1 complex activities) and reverse zymography (PAI-1 activity). The second panel marked CE is a longer incubation of the gel shown in the top panel.

## Claims

1. Use of 8-Prenylflavanones of the general formula I: 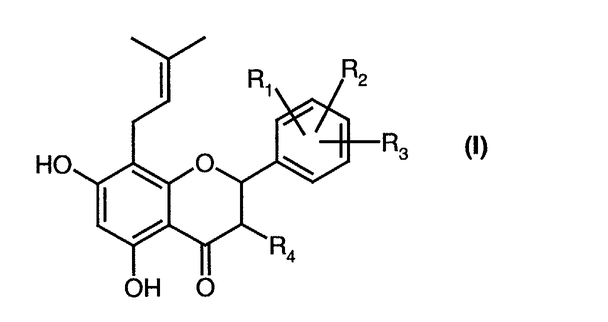 wherein
R₁ designates a hydrogen atom, a hydroxyl group in position 2', 3' or 4', a methoxy group in position 2', 3' or 4' or an ethoxy group in position 3' or 4',
R₂ designates a hydrogen atom, a hydroxyl group in position 3', 4', 5' or 6', a methoxy group in position 3' or 4' or an ethoxy group in position 5',
R₃ designates a hydrogen atom, a hydroxyl group in position 4', 5' or 6' or a methoxy group in position 4', 5' or 6',
R₄ designates a hydrogen atom or a hydroxyl group,
for the preparation of a pharmaceutical 'composition for the treatment or prevention of angiogenesis-related diseases and for the treatment or prevention of thrombotic disorders.

2. Use according to claim 1 wherein the 8-Prenylflavanone is 8-Prenylnaringenin.

3. Use according to claim 1 wherein the angiogenesis-related disease is cancer.

4. A pharmaceutical composition comprising one or more 8-Prenylflavanones of the general formula I and at least one pharmaceutically acceptable excipient for the prevention and treatment of angiogenesis-related diseases.

5. Pharmaceutical composition according to claim 4 wherein the 8-Prenylflavanone is 8-Prenylnaringenin

6. Pharmaceutical composition according to claim 4 or 5 wherein the angiogenesis-related disease is cancer.

7. A pharmaceutical composition comprising one or more 8-Prenylflavanones of the general formula I and at least one pharmaceutically acceptable excipient for the prevention and treatment of thrombotic disorders.

8. Pharmaceutical composition according to claim 7 wherein the 8-Prenylflavanone is 8-Prenylnaringenin
